# EUROPEAN PATENT APPLICATION

(11) **EP 3 978 122 A1**
(43) Date of publication of application: **06.04.2022**
(21) Application number: 21199486.8
(22) Date of filing: 28.09.2021
(51) Int. Cl.: B01L 3/00

(54) **DETECTION CHIP, NUCLEIC ACID DETECTION KIT, AND NUCLEIC ACID DETECTION DEVICE**

(30) Priority: 30.09.2020 US 202063085385 P; 30.09.2020 US 202063085368 P; 31.05.2021 CN 202110604893
(71) Applicant: iCare Diagnostics International Co. Ltd., New Taipei 236 (TW); Century Technology (Shenzhen) Corporation Limited, Shenzhen, Guandong (CN)
(72) Inventor: HUANG, HUNG-YUN, New Taipei (TW); HUANG, CHIH LUN, New Taipei (TW); HSIEH, JEN-CHIN, New Taipei (TW); LEE, CHUN-CHI, New Taipei (TW); WENG, YU-FU, New Taipei (TW)
(74) Representative: Zaboliene, Reda

(57) **Abstract**

A detection chip (100a, 100b, 100c, 100d, 100e) includes a first cover plate (1), a second cover plate (2), a conductive portion (3), and two first driving electrodes (4).

The conductive portion (3) is connected to the first cover plate (1) and the second cover plate (2). The first cover plate (1), the conductive portion (3), and the second cover plate (2) cooperatively define a channel (5) for carrying a microbead (7). The channel (5) includes a flow path (6). The two first driving electrodes (4) are electrically connected to the conductive portion (3) to energize the conductive portion (3). When the conductive portion (3) is energized, a driving force is generated to drive the microbead (7) away from a sidewall of the conductive portion (3) to the flow path (6). A nucleic acid detection kit (200) with the detection chip (100a, 100b, 100c, 100d, 100e), and a nucleic acid detection device (300) with the nucleic acid detection kit (200) are also disclosed.

## Description

### FIELD

The subject matter relates to nucleic acid detection devices, and more particularly, to a detection chip, a nucleic acid detection kit with the detection chip, and a nucleic acid detection device with the nucleic acid detection kit.

### BACKGROUND

Molecular diagnosis, morphological detection, and immunological detection are mostly carried out in a microfluidic chip. The microfluidic chip includes a channel for carrying a microbead. The microbead performs a polymerase chain reaction (PCR) amplification reaction in the channel. However, the microbead may be absorbed on a sidewall of the channel during the detection process, so that the microbead cannot move in a flow path that results in a failure of the PCR amplification reaction. Therefore, there is room for improvement in the art.

### SUMMARY

To overcome the above shortcomings, the present disclosure provides a detection chip, including a first cover plate, a second cover plate, a conductive portion, and two first driving electrodes. The conductive portion includes a first surface and a second surface, the first surface and the second surface are respectively connected to the first cover plate and the second cover plate, the first cover plate, the conductive portion, and the second cover plate cooperatively define a channel configured for carrying a microbead. The channel includes a flow path, the microbead is charged, each of the two first driving electrodes is electrically connected to the conductive portion to energize or de energize the conductive portion, when the conductive portion is energized, a driving force is generated between the conductive portion and the microbead to drive the microbead away from a sidewall of the conductive portion to the flow path to perform a polymerase chain reaction (PCR) amplification reaction.

In some embodiment, the detection chip further comprises a driving unit. The driving unit includes a driving circuit disposed on a surface of the first cover plate close to the second cover plate, a first dielectric layer disposed on a side of the driving circuit close to the second cover plate, a first conductive layer disposed on a surface of the second cover plate close to the first cover plate, and a second dielectric layer disposed on a side of the first conductive layer close to the first cover plate, the first dielectric layer and the second dielectric layer cooperatively define the channel, and the driving circuit defines the flow path.

In some embodiment, the driving circuit includes a plurality of second driving electrodes disposed in an array and a plurality of control electrodes, and each of the plurality of second driving electrodes is electrically connected to a corresponding one of the plurality of control electrodes.

In some embodiment, each of the two first driving electrodes is disposed between the first surface and the first cover plate or between the first surface and the second cover plate, and each of the two first driving electrodes is electrically connected to the conductive portion.

In some embodiment, two first grooves are defined on a surface of the first cover plate corresponds to the first surface, each of the two first driving electrodes is embedded in a corresponding one of the two first grooves, the first surface of the conductive portion includes two first convex blocks, each of the two first convex blocks is embedded in a corresponding one of the first grooves and electrically connected to the first driving electrode in the first groove.

In some embodiment, a first groove is disposed on a surface of the first cover plate corresponds to the first surface, a second groove is disposed on a surface of the second cover plate corresponds to the second surface, the two first driving electrodes are respectively embedded in the first groove and the second groove, the first surface corresponds to the first groove and includes a first convex block, the second surface corresponds to the second groove and includes a second convex block. The first convex block is embedded in the first groove and electrically connect to the first driving electrode in the first groove, and the second convex block is embedded in the second groove and electrically connected to the first driving electrode in the second groove.

In some embodiment, two third grooves are disposed on the conductive portion, and each of the two first driving electrodes is embedded in one of the two third groove and electrically connected to the conductive portion.

In some embodiment, the conductive portion includes a conductive body and a second conductive layer disposed on a surface of the conductive body close to the channel, the second conductive layer is electrically connected to the two first driving electrodes.

The present disclosure further provides a nucleic acid detection kit, including the above detection chip, a kit body, and a connector. The detection chip is disposed in the kit body and electrically connected to the connector.

The present disclosure further provides a nucleic acid detection device, including the above nucleic acid detection kit and a host. The host includes a mounting groove, the nucleic acid detection kit is detachably disposed in the mounting groove.

The detection chip of the present disclosure has a simple structure, which is portable, flexible, and convenient, and is easy to assemble. The cooperation between the conductive portion and the microbead can drive the microbead away from the sidewall of the channel to the flow path, so that prevents a failure of the PCR amplification reaction.

### BRIEF DESCRIPTION OF THE DRAWINGS

Implementations of the present technology will now be described, by way of example only, with reference to the attached figures.
FIG. 1 is a diagrammatic view of an embodiment of a detection chip according to the present disclosure.
FIG. 2 is a cross-sectional view of an embodiment of a detection chip according to the present disclosure.
FIG. 3 is a diagrammatic view of an embodiment of a flow path in the detection chip according to the present disclosure.
FIG. 4 is a diagrammatic view of the detection chip in FIG. 2 when a microbead is absorbed on a sidewall of a channel.
FIG. 5 is a diagrammatic view of the detection chip in FIG. 2 when the microbead is driven away from the sidewall.
FIG. 6 is a cross-sectional view of another embodiment of a detection chip according to the present disclosure.
FIG. 7 is a cross-sectional view of another embodiment of a detection chip according to the present disclosure.
FIG. 8 is a cross-sectional view of another embodiment of a detection chip according to the present disclosure.
FIG. 9 is a cross-sectional view of another embodiment of a detection chip according to the present disclosure.
FIG. 10 is a cross-sectional view of another embodiment of a detection chip according to the present disclosure.
FIG. 11 is a diagrammatic view of an embodiment of a nucleic acid detection kit according to the present disclosure.
FIG. 12 is a diagrammatic view of an embodiment of a nucleic acid detection device according to the present disclosure.

### DETAILED DESCRIPTION

It will be appreciated that for simplicity and clarity of illustration, where appropriate, reference numerals have been repeated among the different figures to indicate corresponding or analogous components. In addition, numerous specific details are set forth in order to provide a thorough understanding of the embodiments described herein. However, it will be understood by those of ordinary skill in the art that the embodiments described herein can be practiced without these specific details. In other instances, methods, procedures, and components have not been described in detail so as not to obscure the related relevant feature being described- Also, the description is not to be considered as limiting the scope of the embodiments described herein. The drawings are not necessarily to scale, and the proportions of certain parts may be exaggerated to better illustrate details and features of the present disclosure.

The term "comprising," when utilized, means "including, but not necessarily limited to"; it specifically indicates open-ended inclusion or membership in the so-described combination, group, series, and the like.

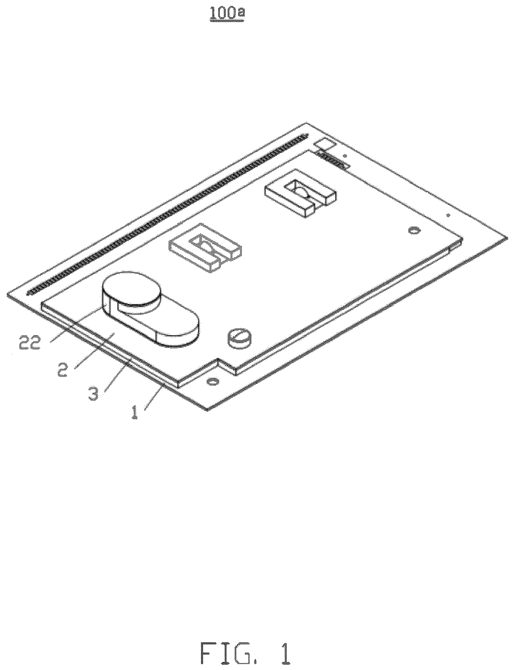

FIGS. 1 to 3 illustrate a detection chip 100a, which includes a first cover plate 1, a second cover plate 2, a conductive portion 3, and two first driving electrodes 4. The conductive portion 3 includes a first surface 31 and a second surface 32. The first surface 31 and the second surface 32 are connected to the first cover plate 1 and the second cover plate 2, respectively. The first cover plate 1, the conductive portion 3, and the second cover plate 2 cooperatively define a channel 5 for carrying a solution to be detected. The solution to be detected exists in the channel 5 in a form of microbead 7. The channel 5 includes a flow path 6. The microbead 7 has some conductivity. Each of the two first driving electrodes 4 is electrically connected to the conductive portion 3 to energize or de energize the conductive portion 3. When the conductive portion 3 is energized, a driving force "f" is generated between the conductive portion 3 and the microbead 7 to drive the microbead 7 away from a sidewall of the conductive portion 3 to the flow path 6. The microbead 7 includes a nucleic acid sample. The detection chip 100a is configured to perform a PCR amplification reaction.

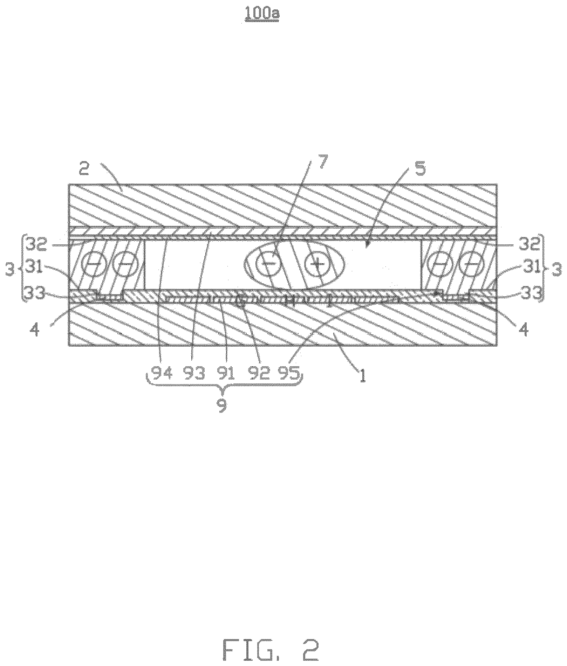

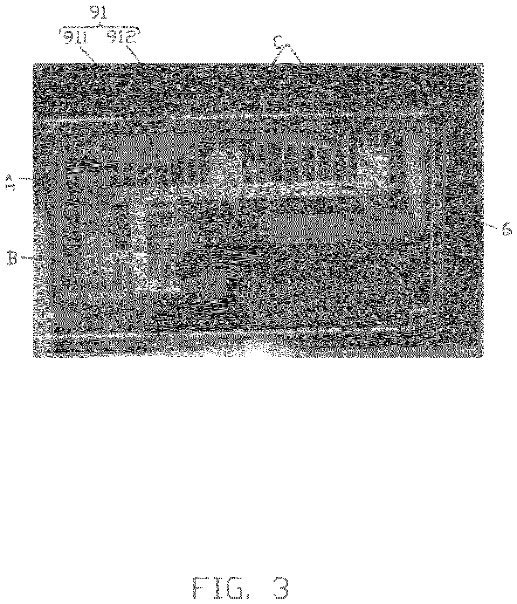

Referring to FIGS. 2 and 3, the detection chip 100a further includes a driving unit 9. The driving unit 9 includes a driving circuit 91 disposed on a surface of the first cover plate 1 close to the second cover plate 2, a first dielectric layer 92 disposed on a side of the driving circuit 91 close to the second cover plate 2, a first conductive layer 93 disposed on a surface of the second cover plate 2 close to the first cover plate 1, and a second dielectric layer 94 disposed on a side of the first conductive layer 93 close to the first cover plate 1. The first dielectric layer 92 and the second dielectric layer 94 cooperatively define the channel 5. The driving circuit 91 defines the flow path 6. The mierobead 7 can be driven by energizing or de energizing the driving circuit 91 to move along the flow path 6 in the channel 5.

Referring to FIGS. 2 and 3, the driving circuit 91 includes a plurality of second driving electrodes 911 disposed in an array and a plurality of control electrodes 912. Each of the second driving electrodes 911 is electrically connected to a corresponding one of the control electrodes 912. In an embodiment, the driving circuit 91 is a thin film transistor (TFT) driving circuit. The microbead 7 can be driven to move on the flow path 6 by the driving circuit 24 due to dielectric wetting principle (EWOD). Due to the EWOD principle, a circuit between one of the second driving electrodes 911 and the first conductive layer 93 can be selectively energized to change wetting characteristics between the microbead 7 and the first dielectric layer 92, and between the microbead 7 and the second dielectric layer 94, and further to control the microbead 7 to move along the flow path 6. Referring to FIG. 2, the second driving electrodes 911 include a driving electrode "I", a driving electrode "H", and a driving electrode "G". The microbead 7 moves on the driving electrode "I", the driving electrode "H", and the driving electrode "G". When the microbead 7 is on the driving electrode "H", a voltage "Vd" is applied between the driving electrode "G" and the first conductive layer 93, and a circuit between the driving electrode "H" and the first conductive layer 93 is disconnected. At this time, the wetting characteristics between the microbead 7 and the first dielectric layer 92, and between the microbead 7 and the second dielectric layer 94 arc changed, so that a liquid-solid contact angle between the driving electrode "H" and microbead 7 becomes larger, and a liquid-solid contact angle between the driving electrode "G" and microbead 7 becomes smaller to promote the movement of microbead 7 from the driving electrode "H" to the driving electrode "G".

In an embodiment, each of the first dielectric layer 92 and the second dielectric layer 94 is insulated and are hydrophobic layer. The first dielectric layer 92 and the second dielectric layer 94 can make the microbead 7 move smoothly along the flow path 6, and avoid a breakage of the microbead 7 during movement.

In an embodiment, each of the first dielectric layer 92 and the second dielectric layer 94 may be, but is not limited to, a polytetrafluoroethylene coating.

In an embodiment, referring to FIG. 2, the driving circuit 91 may be formed on the surface of the first cover plate 1 by metal etching or electroplating.

In an embodiment, referring to FIG. 3, the control electrodes 912 are integrated at an edge of the first cover plate 1. An electrical connection between the detection chip 100a and a connector (not shown) is realized by inserting the side of the first cover plate 1 with the control electrodes 912 into an interface of the connector.

Referring to FIG. 3, the driving circuit 91 can be divided into a plurality of areas according to different purposes, includes sample adding area "A", a reagent storage area "B", a plurality of PCR amplification areas "C", and an outlet area "D". A sampling groove 22 corresponds to the sample adding area "A" is disposed on the second cover plate 2. The sampling groove 22 is connected to the sample adding area "A". The microbead 7 is added in the sampling area "A" through the sampling groove 22. The reagent storage area "B" is configured to store fluorescent reagent (such as a fluorescent dye or a fluorescent probe). The microbead 7 undergoes the PCR amplification reaction in the PCR amplification areas "C". The number of the PCR amplification areas "C" can be set according to an actual detection requirement.

Referring to FIGS. 2 and 3, after the microbead 7 enters the sampling area "A", the microbead 7 moves to the nucleic acid amplification areas "C" and undergoes the PCR amplification reaction to form an amplified product. When the PCR amplification reaction is completed, the amplified product is moved to the reagent storage area "B" and mixed with the fluorescent reagent to obtain a mixture. The mixture then enters the next step (such as electrophoretic detection). In an embodiment, the microbead 7 has positive charges. During the movement, if the microbead 7 is adsorbed on the sidewall of the conductive portion 3 due to maloperation or electrostatic attraction, the negative charges of the conductive portion 3 neutralizes the positive charges in the microbead 7 and further transfer to the microbead 7, so that a driving force will be formed between the microbead 7 with negative charges and the conductive portion 3. The microbead 7 is driven away from the sidewall of the conductive portion 3 by the driving force and returns to the flow path 6 again to ensure the PCR amplification reaction. The cooperation between the conductive portion 3 and the microbead 7 can drive the microbead 7 away from the sidewall of the channel 5 to the flow path 6, so that avoids a failure of the PCR amplification reaction.

The first driving electrodes 4 and the conductive portion 3 can be electrically connected to each other in a variety of ways, which is described as follows.

In an embodiment, referring to FIG. 2, each of the two first driving electrodes 4 is disposed between the first cover plate 1 and the first surface 31 of the conductive portion 3, and is electrically connected to the conductive portion 3. To ensure the sealing effect of the channel 5, a sealing material (not shown) is filled between the first surface 31 and a surface of the first cover plate 1 close to the second cover plate 2. The connection between the first driving electrodes 4 and a power supply can be easily realized.

In an embodiment, referring to FIG. 2, the first dielectric layer 92 defines two openings 95. Each of the first driving electrodes 4 is embedded in one of the openings 95. Two opposite surfaces of each of the first driving electrodes 4 are connected to the first surface 31 and the surface of the first cover plate 1 close to the second cover plate 2. The first dielectric layer 92 fills a gap between the first surface 31 and the first cover plate 1 to improve a sealing effect of the channel 5.

In an embodiment, referring to FIG. 2, two bumps 33 are disposed on the first surface 31 of the conductive portion 3. Each of the bumps 33 is embedded in one of the openings 95 and is electrically connected to the first driving electrode 4. Cooperation of the bump 33 and the opening 95 can improve the connection stability between the first driving electrode 4 and the first surface 31.

In an embodiment, each of the first driving electrodes 4 may be but is not limited to an electrode sheet.

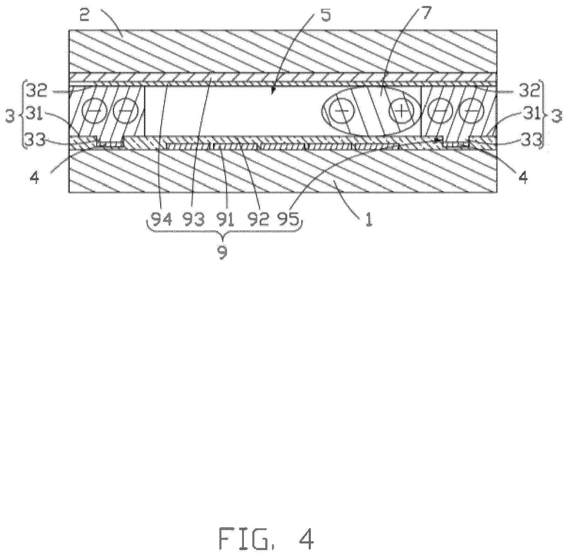

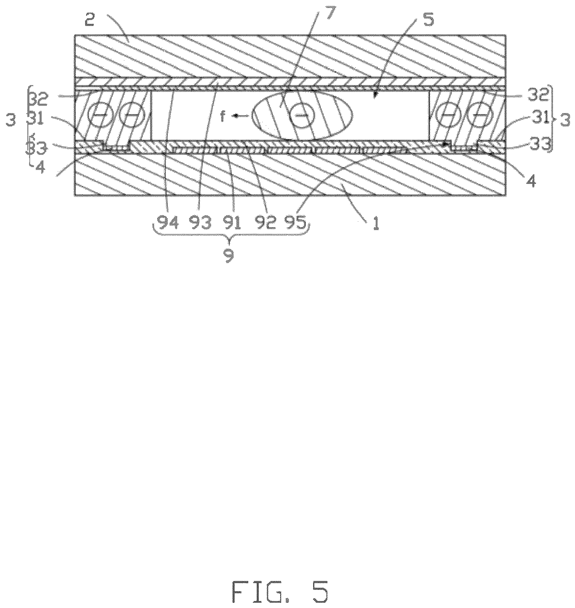

The two first driving electrodes 4 are connected to a positive pole and a negative pole of the power supply to energize the conductive portion 3 to have a negative voltage. The microbead 7 has positive charges. Referring to FIGS. 3 and 4, an electrostatic force between the conductive portion 3 and the microbead 7 adsorbs the microbead 7 from the flow path 6 to the sidewall of the conductive portion 3 close to the channel 5. Then, the negative charges of the conductive portion 3 neutralizes the positive charges in the microbead 7 and further transfer to the microbead 7, so that a driving force will be formed between the microbead 7 and the conductive portion 3. Referring to FIGS. 3 and 5, the microbead 7 is driven away from the sidewall of the conductive portion 3 by the driving force and returns to the flow path 6 again to ensure the PCR amplification reaction.

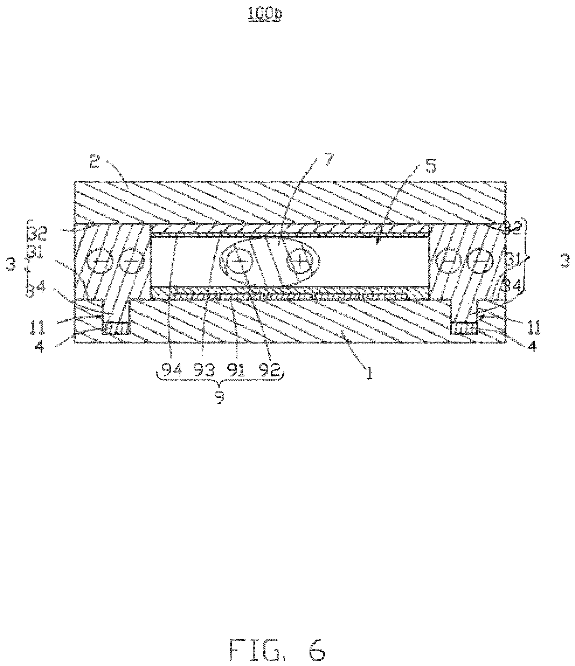

FIG. 6 illustrates another detection chip 100b. Two first grooves 11 are disposed on a surface of the first cover plate 1 corresponds to the first surface 31. Each of the two first driving electrodes 4 is embedded in a corresponding one of the first grooves 11. The first surface 31 of the conductive portion 3 comprises two first convex blocks 34. Each of the two first convex blocks 34 is embedded in a corresponding one of the two first grooves 11 and electrically connected to the first driving electrode 4 in the first groove 11. Cooperation of the first groove 11 in the first cover plate 1 and the first convex block 34 on the first surface 31 can improve the connection stability between the first driving electrode 4 and the conductive portion 3. The connection of the first cover plate 1 and the conductive portion 3 can further improve the sealing effect of the channel 5. Each of the first driving electrodes 4 may be but is not limited to an electrode sheet.

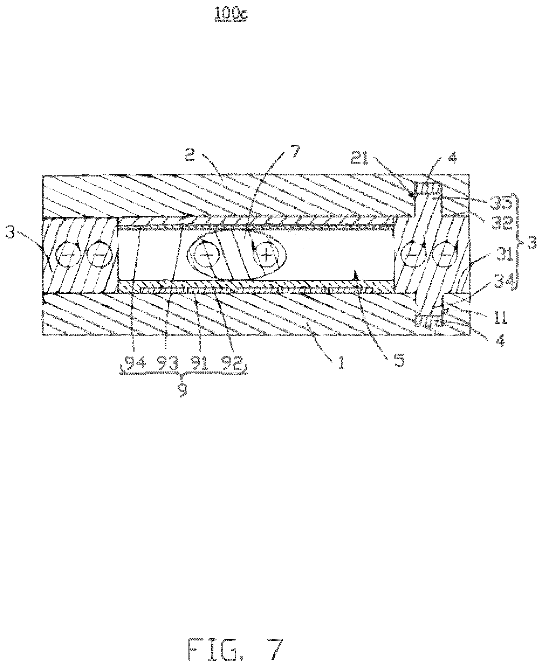

FIG. 7 illustrates another detection chip 100c. A first groove 11 is disposed on a surface of the first cover plate 1 corresponds to the first surface 31. A second groove 21 is disposed on a surface of the second cover plate 2 corresponds to the second surface 32. The first driving electrodes 4 are respectively embedded in the first groove 11 and the second groove 21. The first surface 31 corresponds to the first groove 11 and comprises a first convex block 34. The second surface 32 corresponds to the second groove 21 and comprises a second convex block 35. The first convex block 34 is embedded in the first groove 11 and electrically connected to the first driving electrode 4 in the first groove 11. The second convex block 35 is embedded in the second groove 21 and comprises electrically connected to the first driving electrode 4 in the second groove 21. Cooperation of the first groove 11, the second groove 21, the first convex block 34, and the second convex block 35 can improve the connection stability between the first driving electrodes 4 and the conductive portion 3. The connection of the first cover plate 1, the second cover plate 2, and the conductive portion 3 can further improve the sealing effect of the channel 5. Each of the first driving electrodes 4 may be but is not limited to an electrode sheet.

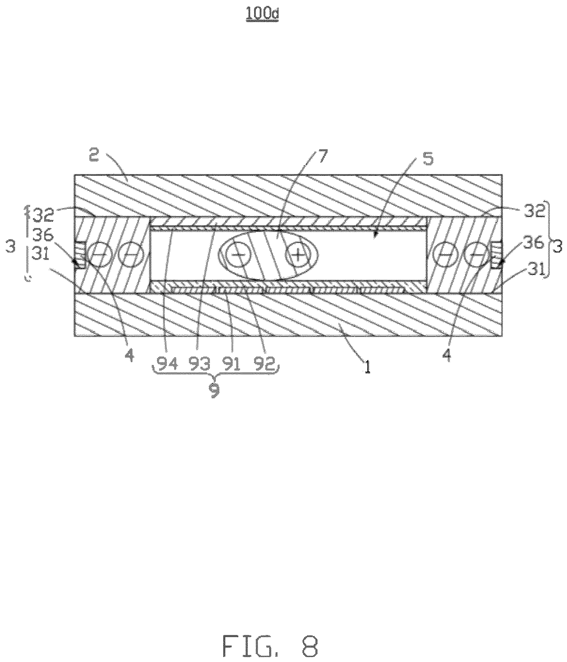

FIG. 8 illustrates another detection chip 100d. Two third grooves 36 are disposed on the conductive portion 3. Each of the first driving electrodes 4 is embedded in a third groove 36 and electrically connected to the conductive portion 3. The third groove 36 arranges on the conductive portion 3 is simple to form and convenient for the assembly of the detection chip 100d. A position of the third groove 36 on the conductive portion 3 can be set according to an actual need. An opening of the third groove 36 can be disposed on the first surface 31 and / or the second surface 32, or on a sidewall of the conductive portion 3 away from the channel 5.

In an embodiment, referring to FIG. 8, the opening of the third groove 36 can be disposed on the sidewall of the conductive portion 3 away from the channel 5, so that the first driving electrode 4 in the third groove 36 is easy to connect to the power supply. Each of the first driving electrodes 4 may be but is not limited to an electrode sheet.

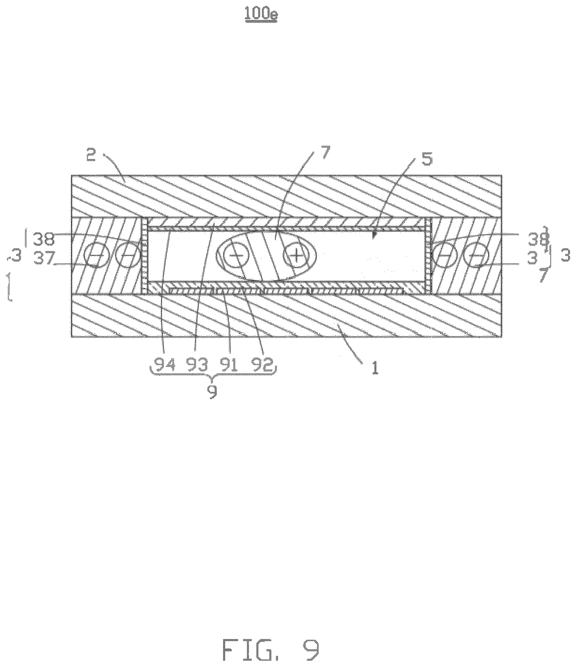

FIG. 9 illustrates another detection chip 100c. The conductive portion 3 includes a conductive body 37 and a second conductive layer 38 disposed on a surface of the conductive body 37 close to the channel 5. The second conductive layer 38 is electrically connected to the two first driving electrodes 4. On a premise of ensuring the sealing effect of the channel 5 and the electrical connection between the second conductive layer 38 and the power supply, the first driving electrode 4 can be electrically connected to the second conductive layer 38 in any form.

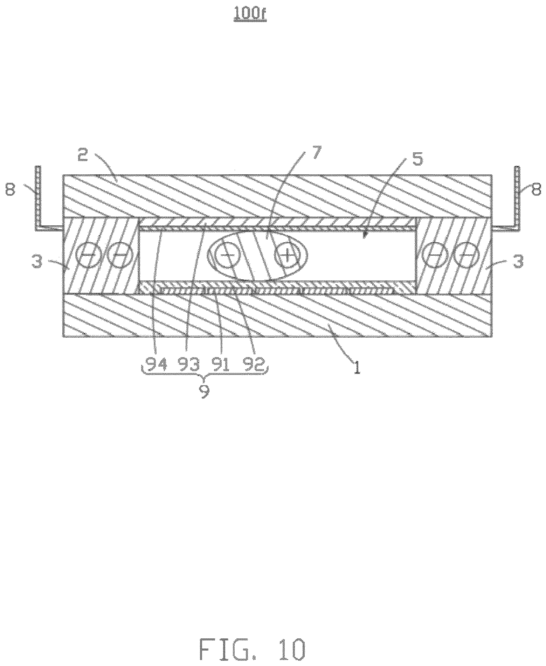

FIG. 10 illustrates another detection chip 100f. The first driving electrode 8 is a long strip electrode with a certain length diameter ratio, such as a needle electrode or rod electrode. Two ends of the first driving electrode 8 are connected to the conductive portion 3 and the power supply, respectively. The first driving electrode 8 is easy to connect to the conductive portion 3 and the power supply, which can reduce a forming difficulty of the detection chip 100f.

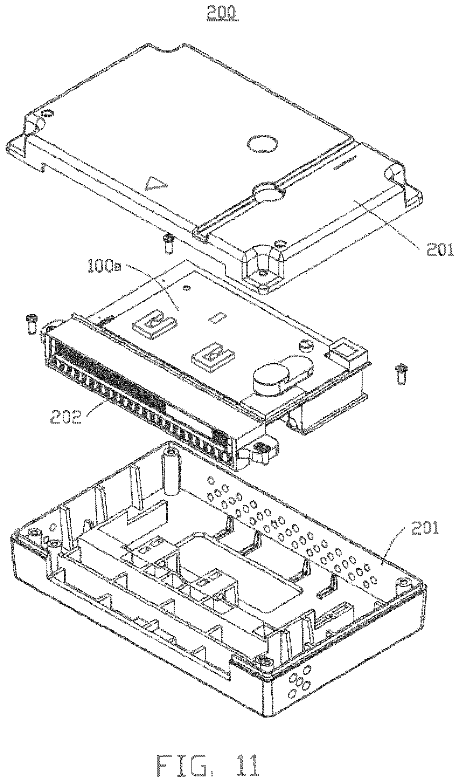

FIG. 11 illustrates a nucleic acid detection kit 200, which includes a kit body 201, the detection chip 100a (or 100b, 100c, 100d, 100e, 100f), and a connector 202. The detection chip 100a is disposed in the kit body 201. The detection chip 100a is electrically connected to the connector 302.

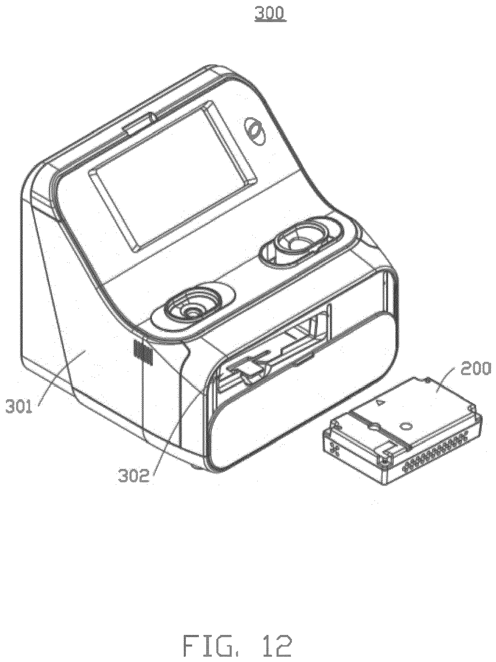

FIG. 12 illustrates a nucleic acid detection device 300, which includes a host 301 and the nucleic acid detection kit 200. The host 301 includes a mounting groove 302. The nucleic acid detection kit 200 is detachably disposed in the mounting groove 302.

With the above configuration, the detection chip has a simple structure, which is portable, flexible, and convenient, and is easy to assemble. The cooperation between the conductive portion 3 and the microbead 7 can drive the microbead 7 away from the sidewall of the channel 5 to the flow path 6, so that prevents a failure of the PCR amplification reaction.

The embodiments shown and described above are only examples. Even though numerous characteristics and advantages of the present technology have been set forth in the foregoing description, together with details of the structure and function of the present disclosure, the disclosure is illustrative only, and changes may be made in the detail, including in matters of shape, size and arrangement of the parts within the principles of the present disclosure, up to and including, the full extent established by the broad general meaning of the terms used in the claims.

## Claims

1. A detection chip (100a, 100b, 100c, 100d, 100e), **characterized in that**, comprising:
a first cover plate (1);
a second cover plate (2);
a conductive portion (3); and
two first driving electrodes (4);
the conductive portion (3) comprises a first surface (31) and a second surface (32), the first surface (31) and the second surface (32) are respectively connected to the first cover plate (1) and the second cover plate (2), the first cover plate (1), the conductive portion (3), and the second cover plate (2) cooperatively define a channel (5) configured for carrying a microbead (7);
the channel (5) comprises a flow path (6), the microbead (7) is charged, each of the two first driving electrodes (4) is electrically connected to the conductive portion (3) to energize or de energize the conductive portion (3), when the conductive portion (3) is energized, a driving force is generated between the conductive portion (3) and the microbead (7) to drive the microbead (7) away from a sidewall of the conductive portion (3) to the flow path (6) to perform a polymerase chain reaction (PCR) amplification reaction.

2. The detection chip (100a) of claim 1, **characterized in that**, the detection chip (100a) further comprises a driving unit (9), the driving unit (9) comprises a driving circuit (91) disposed on a surface of the first cover plate (1) close to the second cover plate (2), a first dielectric layer (92) disposed on a side of the driving circuit (91) close to the second cover plate (2), a first conductive layer (91) disposed on a surface of the second cover plate (2) close to the first cover plate (1), and a second dielectric layer (2) disposed on a side of the first conductive layer (93) close to the first cover plate (1), the first dielectric layer (91) and the second dielectric layer (92) cooperatively define the channel (5), and the driving circuit (91) defines the flow path (6).

3. The detection chip (100a) of claim 2, **characterized in that**, the driving circuit (91) comprises a plurality of second driving electrodes (911) disposed in an array and a plurality of control electrodes (912), and each of the plurality of second driving electrodes (911) is electrically connected to a corresponding one of the plurality of control electrodes (912).

4. The detection chip (100a) of claim 1, **characterized in that**, each of the two first driving electrodes (4) is disposed between the first surface (31) and the first cover plate (1) or between the first surface (31) and the second cover plate (2), and each of the two first driving electrodes (4) is electrically connected to the conductive portion (3).

5. The detection chip (100b) of claim 1, **characterized in that**, two first grooves (11) are defined on a surface of the first cover plate (1) corresponds to the first surface (31), each of the two first driving electrodes (4) is embedded in a corresponding one of the two first grooves (11), the first surface (31) of the conductive portion (3) comprises two first convex blocks (34), each of the two first convex blocks (34) is embedded in a corresponding one of the first grooves (11) and electrically connected to the first driving electrode (4) in the first groove (11).

6. The detection chip (100c) of claim 1, **characterized in that**, a first groove (11) is disposed on a surface of the first cover plate (1) corresponds to the first surface (31), a second groove (21) is disposed on a surface of the second cover plate (2) corresponds to the second surface (32), the two first driving electrodes (4) are respectively embedded in the first groove (11) and the second groove (21), the first surface (31) corresponds to the first groove (11) and comprises a first convex block (34), the second surface (32) corresponds to the second groove (21) and comprises a second convex block (35);
the first convex block (34) is embedded in the first groove (11) and electrically connect to the first driving electrode (4) in the first groove (11), and the second convex block (35) is embedded in the second groove (21) and electrically connected to the first driving electrode (4) in the second groove (21).

7. The detection chip (100d) of claim 1, **characterized in that**, two third grooves (36) are disposed on the conductive portion (3), and each of the two first driving electrodes (4) is embedded in one of the two third groove (36) and electrically connected to the conductive portion (3).

8. The detection chip (100e) of claim 1, **characterized in that**, the conductive portion (3) comprises a conductive body (37) and a second conductive layer (38) disposed on a surface of the conductive body (37) close to the channel (5), the second conductive layer (38) is electrically connected to the two first driving electrodes (4).

9. A nucleic acid detection kit (200), **characterized in that**, comprising:
a detection chip (100a, 100b, 100c, 100d, 100e) of any one of claims 1 to 8;
a kit body (201); and
a connector (302), the detection chip (100a, 100b, 100c, 100d, 100e) disposed in the kit body (201) and electrically connected to the connector (302).

10. A nucleic acid detection device (300), **characterized in that**, comprising:
a nucleic acid detection kit (200) of claim 9; and
a host (301), the host (301) comprises a mounting groove (302), the nucleic acid detection kit (201) is detachably disposed in the mounting groove (302).
